# EUROPEAN PATENT APPLICATION

(11) **EP 4 756 829 A1**
(43) Date of publication of application: **10.06.2026**
(21) Application number: 25220082.9
(22) Date of filing: 02.12.2025
(51) Int. Cl.: G16H 30/20, G16H 40/40, G16H 40/63, G16H 30/40

(54) **VENDOR-AGNOSTIC AUTOMATED IMAGE QUALITY CHECK FOR REMOTE COMMAND CENTER**

(30) Priority: 05.12.2024 US 202463728478 P; 17.12.2024 EP 24220608
(71) Applicant: Koninklijke Philips N.V., 5656 AE Eindhoven (NL)
(72) Inventor: TELLIS, Ranjith Naveen, 5656AG Eindhoven (NL); AMTHOR, Thomas Erik, 5656AG Eindhoven (NL); DALAL, Sandeep Madhukar, 5656AG Eindhoven (NL); CHADUVULA, Siva Chaitanya, 5656AG Eindhoven (NL)
(74) Representative: Philips Intellectual Property & Standards

(57) **Abstract**

Disclosed is a computer-implemented method (100) for performing image quality checks using a control system (200), the method comprising by the control system (200): receiving (102) screen capture medical image data (202) of a user interface (204) of a medical imaging system (206); extracting (104) exam context information (208) and medical images (210) from the screen capture medical image data (202); based on the extracted exam context information (208), selecting (106) an image evaluation protocol (212), wherein the image evaluation protocol (212) comprises at least one image metric (214); executing (108) the image evaluation protocol (212) on the extracted medical images (210), resulting in at least one value of the at least one image metric (214); and based on the at least one value of the at least one image metric (214), providing (110) a control signal (216) to the medical imaging system (206).

## Description

### FIELD OF THE INVENTION

The invention relates to the field of medical imaging, in particular to providing assistance to local operators of a medical imaging system, wherein the assistance particularly comprises image quality checks of medical images obtained from the medical imaging system.

### BACKGROUND OF THE INVENTION

Medical images of a subject acquired by a medical imaging system are commonly required to be reviewed by a local operator operating the medical imaging system or another expert possessing relevant domain expertise located at a site of the medical imaging system. The local operator may evaluate the quality of the medical images, while the subject is still present at the site of the medical imaging system. Should the local operator identify defects in the medical images, the medical imaging examination may be repeated, after modification of exam parameters of the medical imaging system, and further medical images may be acquired as needed. For example, further medical images may need to be acquired in case an anatomical structure to be imaged was not fully covered by the medical images, or the medical images display artifacts, for example motion artifacts arising due to movements of the subject.

The patent application WO2014113530A1 describes a scan completeness auditing system for use with an imaging console in screening a volume of tissue comprising a position tracking system configured to track and record a position of a manual imaging probe. The position tracking system comprises a plurality of cameras adapted to couple to the manual imaging probe and configured to provide position data for the manual imaging probe. The scan completeness auditing system includes a receiver comprising a controller-configured to electronically receive position data for the manual ultrasonic imaging probe from the position tracking system and to electronically receive and record a first scan sequence comprising a first set of scanned images representing cross-sections of the tissue from the manual imaging probe. The controller can be configured to compute an image-to-image spacing between successive images within the first scan sequence and to determine whether the computed image-to-image spacing exceeds a maximum limit. An alert when the computed image-to-image spacing exceeds the maximum limit.

WO2024000059A1 discloses systems and methods for generating and transmitting media data feeds location-based privacy settings. Embodiments generate, store, and reference location-based privacy settings that instruct the computing device to generate obfuscations in the media data. The obfuscations may include computer-generated or user gesture-generated obfuscation regions that instruct the computing device to apply the obfuscations to portions of the video feed.

### SUMMARY OF THE INVENTION

The invention describes a method control system, computer program and non-transitory computer-readable medium for performing image quality checks. The invention is defined by the independent claims. The dependent claims define advantageous embodiments.

In one aspect, the disclosure describes a computer-implemented method for performing image quality checks using a control system. The method comprises receiving screen capture medical image data of a user interface of a medical imaging system by the control system. The method further comprises extracting exam context information and medical images from the screen capturing medical image data by the control system. Based on the extracted exam context information, the method further comprises selecting an image evaluation protocol, wherein the image evaluation protocol comprises at least one image metric. The method further comprises executing the image evaluation protocol on the extracted medical images by the control system, resulting in at least one value of the at least one image metric. Based on the at least one value of the at least one image metric, the method further comprises providing a control signal to the medical imaging system by the control system.

In another aspect, the disclosure describes a control system configured for performing image quality checks. The control system comprises a memory storing machine executable instructions, wherein execution of the machine executable instructions causes the control system to receive screen capture medical image data of a user interface of a medical imaging system by the control system. Execution of the machine executable instructions further causes the control system to extract exam context information and medical images from the screen to capture medical image data by the control system. Based on the extracted exam context information, execution of the machine executable instructions further causes the control system to select an image evaluation protocol, wherein the image evaluation protocol comprises at least one image metric. Execution of the machine executable instructions further causes the control system to execute the image evaluation protocol on the extracted medical images by the control system, resulting in at least one value of the at least one image metric. Based on the at least one value of the at least one image metric, execution of the machine executable instructions further causes the control system to provide a control signal to the medical imaging system by the control system.

In another aspect, the disclosure describes a computer program product or a non-transitory computer-readable medium, wherein the computer program product comprises machine executable instructions configured for causing a control system to execute the disclosed method.

One advantage may reside in providing an automated image quality check of the acquired medical images via the image evaluation protocol, wherein the image evaluation protocol provides information on the quality of the medical images. This may enable improving of the quality of the medical images without requiring manual intervention, for example by a radiologist or other expert possessing relevant domain expertise, to control the medical imaging system. The automated image quality check may further improve the speed of the image quality check compared to a manual image quality check, in particular when the automated quality check is performed on large datasets of medical images.

Another advantage may reside in enabling an automated control of the medical imaging system by the control system in response to the executing of the image evaluation protocol using the control signal. In particular, said automatic control may be performed while the subject is still at the site of the medical imaging system, reducing the time needed to acquire medical images.

### BRIEF DESCRIPTION OF THE DRAWINGS

In the following, examples are described in greater detail referring to the drawings in which:
Fig. 1 is a flowchart of a method for performing image quality checks using a control system.
Fig. 2 is a block diagram of an exemplary control system for implementing at least part of the present method in accordance with an example of the present subject matter.
Fig. 3A is a schematic diagram of the control system interacting with multiple computer systems of multiple medical imaging systems, wherein a remote computer system comprises the control system.
Fig. 3B is a schematic diagram of the control system interacting with multiple computer systems of multiple medical imaging systems, wherein a cloud-based computer system comprises the control system.
Fig. 3C is a schematic diagram of the control system interacting with multiple computer systems of multiple medical imaging systems, wherein a local computer system of a medical imaging system comprises the control system.
Fig. 4A is a schematic diagram of a user interface of a medical imaging system displaying medical images, as shown on a display interface of a computer system of the medical imaging system.
Fig. 4B is a schematic diagram of screen replications of multiple user interfaces of multiple medical imaging systems displaying medical images, as shown on a display unit of the control system.
Fig. 5 is a block diagram of an exemplary computer system for implementing at least part of the present method in accordance with an example of the present subject matter.

### DETAILED DESCRIPTION OF EMBODIMENTS

The invention will be described with reference to the Figures. It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

In one example, the screen capture medical image data is received from a capture module providing a screen replication of the user interface of the medical imaging system 206.

The capture module enables the control system to receive real time information on a state of the medical imaging system, in particular information on ongoing medical imaging examinations via the information comprised by the screen capture medical image data.

In one example, the control system is configured to provide control functionalities to the user interface of the medical imaging system using the control signal. The extracting of the exam context information and/or the medical images from the screen capture medical image data may preferably be performed using the control signal. The disclosed method may be performed repeatedly, starting with an initial receiving of the screen capture medical image data. Upon repetition, the extracting of the exam context information and/or the medical images from the screen capture medical data may be performed using the control signal. The control signal may be configured to both provide information based on the at least one value of the at least one image metric, while simultaneously performing a further extracting of the medical images and/or the exam context information.

The control functionalities enable the control system to provide information to the medical imaging system via the control signal, said information in particular comprising information obtained from the image evaluation protocol. The control functionalities further enable control of the medical imaging system independent of a local operator at the site of the medical imaging system.

In an embodiment, a method is described, wherein the control signal extracts the context information and/or the medical images, wherein the extracting of the exam context information and/or the medical images from the screen capture medical image data is performed repeatedly using the determined control signal, i.e., the system acquires the capture the medical image data, extracts the context information to select an image evaluation protocol with an image metric resulting with certain values in order to provide a control signal to imaging system, wherein the control signal is used for another round for extraction of medical images, and repeating the cycles until a certain criterion is reached that satisfies e.g., the quality control metric determined by the hospital.

In one example, the exam context information comprises any one of the following: exam type information, wherein the exam type information is descriptive of a medical scanning technique that was used for generating the medical images and/or information descriptive of an anatomical area shown by the medical images; interface configuration information, wherein the interface configuration information is descriptive of a geometric layout of the user interface of the medical imaging system and/or information on positions of the exam context information in the screen capture medical image data; exam progress information; exam parameters, wherein the exam parameters comprise medical imaging system parameters of the medical scanning technique used for generating the medical images.

The exam context information enables a detection of information about the medical imaging system and ongoing medical imaging examinations at the medical imaging system by the control system. The control system may advantageously use the exam context information to determine the control functionalities. Furthermore, the exam context information may advantageously enable a vendor-agnostic control of the medical imaging system by identifying a vendor of the medical imaging system and adapting the control functionalities based on the vendor.

In one example, the control system further comprises an interface control command generator configured to generate the control signal, wherein optionally the control signal comprises interface control commands, the interface control commands comprising a mouse movement command, mouse click command, mouse wheel scrolling command, a keystroke on a keyboard, a text copy command, a text insertion command and/or a screenshot command.

The interface control command generator may enable real time control of the user interface by the control system using the control signal. For example, the control signal enables an automatic extracting of the exam context information and the medical images without a need for manual instructions.

In one example, the extracting of the exam context information comprises extracting text information from the screen capture medical image data using optical character recognition and/or using a neural network, wherein the neural network is configured to detect the text information. Said use of optical character recognition and/or a neural network may advantageously enable an extracting of text information which may not be extracted using the text copy command.

In one example, the medical images are embedded in the screen capture medical image data as displayed images, and wherein the extracting of the medical images comprises any one of: extracting the displayed images as the medical images; generating the control signal comprising a command instructing the user interface of the medical imaging system to perform any one of the following: switch the user interface into an image view mode, wherein the image view mode is configured to display the medical images as the displayed images, remove elements of the user interface occluding the displayed images, iterate through a set of medical images, displaying at least a part of at least one medical image of the set of medical images as the displayed images in each iteration, display the displayed images with a modified resolution, a modified contrast and/or a modified brightness, and extracting the images displayed with the modified resolution, the modified contrast and/or the modified brightness as the medical images.

This may advantageously facilitate the extracting of the medical images and/or the exam context information by enabling a control of the user interface towards a state of the user interface in which any medical images acquired by the medical imaging system may be displayed without occlusions using the control signal. Furthermore, the extracting of the displayed images with the modified resolution, the modified contrast and/or the modified brightness may advantageously result in performance and/or runtime improvements of the image evaluation protocol.

In one example, the selecting of the image evaluation protocol comprises selecting of a subset of suitable image evaluation protocols from a set of image evaluation protocols, the method further comprising determining the subset, the determining comprising at least anyone of the following: restricting the set of image evaluation protocols to the subset, wherein for the image evaluation protocols of the subset the respective metric of the image evaluation protocol is applicable to the medical images based on the exam type information, restricting the set of image evaluation protocols to the subset, wherein for the image evaluation protocols of the subset the respective metric of the image evaluation protocol specifies required exam parameters, wherein the exam parameters of the exam context information satisfy the required exam parameters, restricting the set of image evaluation protocols to the subset based on an average accuracy, an average runtime and/or a computational complexity of the image evaluation protocol.

The restricting to the subset may enable an optimal match of the image evaluation protocol to the medical images, wherein the optimal match may advantageously result in an improved accuracy and/or speed with which the image quality check may be performed.

In one example, the executing of the image evaluation protocol further comprises: detecting image defects by comparing the at least one value of the at least one image metric to at least one threshold value of the image metric and in response to the detecting of the image defects, determining correction instructions, wherein the correction instructions are configured for minimizing the occurrence of image defects, and preferably wherein the correction instructions comprise positioning instructions for positioning a subject imaged by the medical imaging system and/or modified values of the exam parameters.

The automated detecting of the image defects may be advantageous as the control system may detect image defects which may be missed in a manual image quality check. Furthermore, the correction instructions may more precisely minimize the occurrence of the image defects than manually applied corrections.

In one example, the control signal is configured for instructing the user interface of the medical imaging system for executing the correction instructions. Said instructing preferably comprises executing the correction instructions provided by the interface control command generator on the user interface.

The executing of the correction instructions by the user interface due to the control signal may advantageously reduce a time between medical imaging examinations. Furthermore, mistakes which may occur when manually applying the correction instructions on the user interface, for example an unintended switching of digits in a numerical exam parameter, may be avoided.

In one example, the correction instructions are provided automatically after the executing of the image evaluation protocol. For example, the correction instructions may be provided automatically by the interface control command generator after the executing of the image evaluation protocol. The correction instructions may for example automatically correct exam parameters of the medical imaging system by executing interface control commands provided to the medical imaging system by the interface control command generator via the control signal.

The automatic providing of the correction instructions may advantageously reduce the time between repeated medical imaging examinations, thereby reducing a time required to obtain medical images which satisfy the threshold value for the image metrics evaluated by the image evaluation protocol.

In one example, the screen capture medical image data is received repetitively over time, wherein the executing of the image evaluation protocol is performed based on anyone of the following: the exam progress information, a time period since a previous executing of the image evaluation protocol, at least one value of the at least one image metric obtained in the previous executing of the image evaluation protocol, a request by an operator of the control system.

The repetitive receiving of the screen capture medical image data may enable a real time monitoring of a state of the medical imaging system by the control system. The executing of the image evaluation protocol may advantageously be automated and performed at predetermined time points, reducing a need for manual instructions given to the control system. Furthermore, the operator of the control system may request the executing of the image evaluation protocol instead or in addition to the executing at the predetermined time points.

In one example, the medical imaging system is any one of the following: a magnetic resonance imaging system, a computed tomography system, a positron emission tomography system, a single photon emission tomography system, a digital fluoroscopy system, an angiography system and a diagnostic ultrasound system.

According to this example, the method may advantageously be applied to a variety of different medical imaging systems.

The advantages described of the disclosed method equally apply to the disclosed control system and the disclosed computer program product.

In the following, similar elements are denoted by the same reference numerals.

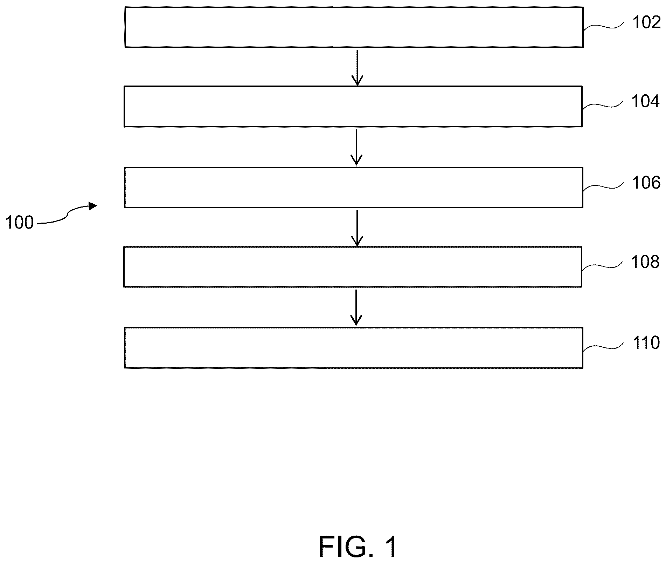

Fig. 1 is a flowchart of a method 100 for performing image quality checks using a control system.

In step 102 of the method 100, the control system receives screen capture medical image data of a user interface of a medical imaging system.

In step 104 of the method 100, the control system extracts exam context information and medical images from the screen capture medical image data.

In step 106 of the method 100, based on the extracted exam context information 208, the control system selects 106 an image evaluation protocol, wherein the image evaluation protocol comprises at least one image metric.

In step 108 of the method 100, the control system executes 108 the image evaluation protocol on the extracted medical images, resulting in at least one value of the at least one image metric. At least one value of the at least one image metric may exemplary be a numerical value, in particular a Boolean value. At least one image metric comprises at least anyone of: a signal-to-noise ratio, SNR, a contrast-to-noise ratio, CNR, an image uniformity, image homogeneity, Hounsfield Units, an image resolution, a coverage and/or metrics quantifying a presence of motion artifacts. The image metrics and the image evaluation protocols used to evaluate the image metrics may differ depending on the medical imaging system.

The image uniformity and/or image homogeneity may exemplarily be quantified by image evaluation protocols evaluating a coefficient of variation describing a ratio of standard deviations to means of pixel intensities of pixels of the medical images, a uniformity index describing a ratio of minimal intensity values to maximum intensity values across the pixels of the medical images.

The coverage may exemplarily be quantified by image evaluation protocols evaluating a coverage ratio of an imaged volume of an anatomical structure within a field of view of the medical imaging system to a total volume of said anatomical structure. Instead or in addition, the coverage may further be quantified by the image evaluation protocols performing landmark detection, for example by detecting vertebrae in medical images of a spine. Furthermore, the coverage may be quantified by the image evaluation protocols performing a bounding box analysis by fitting minimal bounding boxes around the anatomical structure and comparing the minimal bounding box to the field of view.

The presence of motion artifacts may exemplarily be detected by image evaluation protocols performing any one of optical flow analysis, reprojection error analysis, iterative reconstruction techniques, for example adaptive statistical iterative reconstruction (ASIR) or model-based iterative reconstruction (MBIR), edge detection, in particular Sobel or Canny edge detectors to identify blurring, and/or neural networks trained to detect motion artifacts, such as convolutional neural networks (CNNs) or generative adversarial networks (GANs).

In step 110 of the method 100, based on the at least one value of the at least one image metric, the control system provides a control signal to the medical imaging system. Instead or in addition to providing the control signal to the medical imaging system, information contained in the control signal 216 may further be provided by the control system to a computer system of a (remote) operator.

The site of the operator may exemplarily be located in a different room, building or city from the site of the medical imaging system.

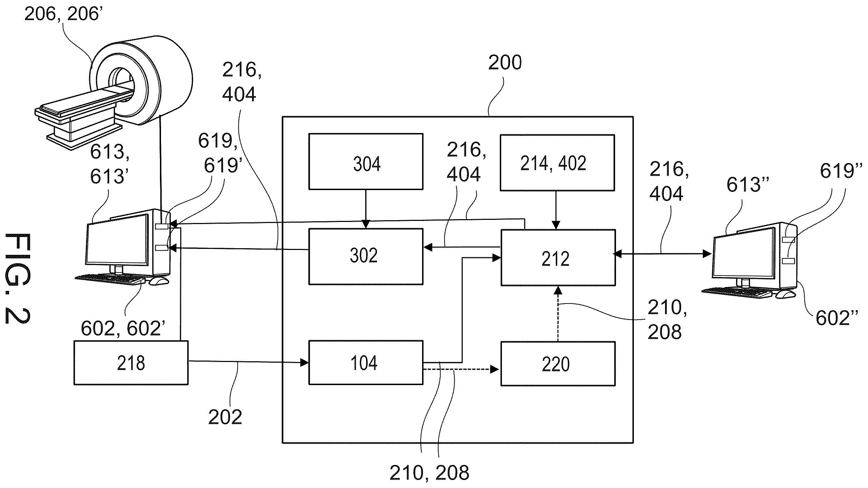

Fig. 1 may be read in conjunction with Fig. 2, wherein Figure 2 is a block diagram of an exemplary control system 200 for implementing at least part of the present method in accordance with an example of the present subject matter.

The exemplary control system 200 is configured to receive screen capture medical image data 202 from a capture module 218 configured to capture the screen capture medical image data 202 from a display 613 of a computer system 602, wherein the computer system 602 is a computer system of a medical imaging system 206. Any examples described in relation to Fig. 2 equally apply to a further computer system 602' of a further medical imaging system 206', wherein the computer system 602' comprises a display unit 613' and I/O-interfaces 619' analogous to the computer system 602. The computer systems 602, 602' of the medical imaging systems 206, 206' may exemplarily comprise external devices 613, 613'. Said external devices 613, 613' may comprise a mouse and/or keyboard configured for controlling the user interfaces of the respective medical imaging systems 206, 206' and/or display units 613, 613' of the respective medical imaging systems. According to this example, the display units 613, 613' are configured to display the respective user interfaces of the medical imaging systems 206, 206'.

According to the example shown in Fig. 2, the control system 200 further comprises an extraction module 104 configured to perform the extracting of the exam context information 208 and the medical images 210 from the screen capture medical image data 202, an image database 220 for storing the exam context information 208 and the medical images 210, and an image evaluation module 212 configured to select and execute the image evaluation protocol.

In one example, the extraction module 104 may provide the exam context information 208 and the medical images 210 to an image evaluation module 212. In an alternative example, the extraction module 104 may be configured to save the exam context information 208 and the medical images 210 in the image database 220, wherein the exam context information 208 and the medical images 210 are provided to the image evaluation module 212 by the image database 220.

The capture module 218 may indicate a component configured to record information displayed on the user interface of the medical imaging system 206. In particular, the capture module 218 may be an external device communicatively coupled to the computer system 602 or the display unit 613, preferably using an HDMI connection. The capture module 218 may preferably be configured to capture a continuous video stream of the user interface. The continuous video stream comprises the screen capture medical image data 202. The capture module 218 is configured to provide the screen capture medical image data 202 to the control system 200, in particular to the extraction module 104. The screen capture medical image data 202 comprises any information displayed on the user interface, wherein said information in particular comprises medical images 210 acquired by the medical imaging system 206.

The control system 200 may further store image metrics 214 and at least one threshold value 402 of the image metrics 214 and provide said image metrics 214 and said at least one threshold value 402 to the image evaluation module 212. The control signal 216 may exemplarily comprise values of the image metrics 214 and be provided by the image evaluation module 212. The control signal may further comprise correction instructions 404 determined based on the values of the image metrics 214. The control signal 216, and in particular the correction instructions 404 may be provided to the computer system 602" of the operator, modified by the operator and returned to the control system 200. The control system is further configured to provide the control signal 216, and in particular the correction instructions 404 to the computer system 602 of the medical imaging system 206. The control signal 216, and in particular the correction instructions 404 may be provided to the computer systems 602, 602" via I/O-interfaces 619, 619', 619". Said I/O interfaces 619, 619', 619" may comprise HDMI interfaces and/or USB interfaces. Said I/O interfaces 619, 619', 619" may further enable the receiving of the screen capture medical image data 202.

The image evaluation module 212 may further be configured to determine which of the external computer systems 602, 602" the control signal 216 is provided to. For example, if a value of an image metric 214 does not exceed the threshold value 402 for said image metric, the control signal 216 may comprise sending the correction instructions 404 only to the computer system 602 of the medical imaging system 206, or to the computer system 602 of the medical imaging system and the computer system 602" of the operator, for example depending on a difference between the value of the image metric 214 and the threshold value 402. Thereby, the operator may be enabled to manually modify the correction instructions 404, provide the modified correction instructions 402 to the control system 200, wherein the control system may provide the correction instructions 402 as the modified correction instructions to the computer system 602 of the medical imaging system 206.

The control system 200 may further comprise an interface control command generator 302 configured to receive the control signal 216, and in particular correction instructions 404, from the image evaluation module 212, determine interface control commands configured for executing the corrections instructions 404, and provide the control signal comprising the correction instructions with the interface control commands to the computer system 602 of the medical imaging system 206.

In one example, the interface control command generator 302 is configured to provide the control functionalities via the control signal 216, in particular via the interface control commands, wherein the providing of the control functionalities comprises sending the interface control commands to the computer systems 602 and executing said interface control commands on the user interface of said computer systems 602, enabling the control system to interact with the user interface of the medical imaging system 206. The control functionalities preferably comprise USB commands sent to the I/O interface 619 of the computer system 602 of the medical imaging system 206 by the control system 200.The control system 200 may further comprise a command database 304, wherein the interface control commands correspond to commands stored as entries in the command database 304. Entries in the command database 304 relate the interface control commands to actions performed on the user interface following an execution of said interface control commands on the user interface. For example, an entry of the command database 304 may relate a scrolling of a mouse wheel to an iterating through medical images in a set of medical images 210 displayed on the user interface. Another entry may relate a scrolling of a mouse wheel to a modification of exam parameters, for example a modification of window width and/or window level parameters in a CT imaging examination. Yet another entry may relate a particular keyboard shortcut to a saving of a medical image 210 or to the opening of a displayed medical image 210 in a higher resolution. Any one of the interface control commands may be combined with mouse movements commands, enabling the interface control commands to be executed with a mouse cursor being positioned at a desired location on the user interface.

The entries in the command database 304 may further comprise information on the relation of particular interface control commands to different types of user interfaces based on the exam context information 208, in particular based on the interface configuration information. The type of user interface may be associated with any entry in the command database 304. For example, different vendors of medical imaging systems 206 may provide similar or identical control functionalities, for example for saving the medical images, wherein the saving is performed using different keystrokes on a keyboard. The interface control commands and the command database 304 thereby enable a vendor-agnostic control of the user interface by the control system 200.

In one example, the medical images 210 may be partial medical images, and the extracting may further comprise stitching, merging or otherwise combining the partial medical images into the medical images 210 by the control system 200. The extracting may further comprise grouping the medical images 210 according to the exam context information 208. For example, grouping may comprise a constructing of at least one three-dimensional medical image 210 from two-dimensional medical images 210. The extracting may preferably comprise extracting the medical images 210 in a predefined file format, the predefined file format for example comprising DICOM, PNG or JPEG. The extracting of the medical images 210 may comprise a storing of the medical images 210 in the image database 220. The image database 220 may further store the exam context information 208 associated with the medical images 210.

Furthermore, the extracting of the medical images 210 may comprise extracting metadata of the medical images 210, the metadata exemplarily comprising a time point at which the medical images were generated by the medical imaging system 206 and/or subject metadata, for example a gender and/or age of a subject, in particular information about the subject being an adult or pediatric subject.

The exam context information 208 may exemplarily comprise information about a medical scanning technique used by the medical imaging system 206 during the medical imaging examination. For example, the medical images 210 may exemplarily result from a magnetic resonance imaging (MRI) medical scanning technique or a computed tomography (CT) medical scanning technique. The medical images 210 comprise anatomical and/or physiological information about the subject. For example, the medical images 210 may display slices of a helical CT examination. In one example, the anatomical and/or physiological information may comprise anatomical and/or physiological information about the brain of the subject, and the medical imaging examination may be performed to detect multiple sclerosis (MS) lesions in the medical images 210 of the brain of the subject.

The exam context information may further comprise a protocol name. The protocol name may for example comprise text information such as "MRI scan", or "Abdominal CT scan" indicating the medical scanning technique and/or an anatomical structure of the body of the subject imaged by the medical imaging examination. Furthermore, the protocol name may comprise text information specific to a particular imaging protocol, for example "MS lesions", indicating that the medical imaging examination is performed to detect multiple sclerosis lesions. The exam context information 208 may further comprise information indicating whether a contrast agent was used, and if so, information indicating a type of contrast agent as well as an amount of the contrast agent used. Moreover, the exam context information 208 may comprise the subject metadata.

The exam context information 208 may exemplarily comprise exam type information, wherein the exam type information is descriptive of the medical scanning technique that was used for generating the medical images 210 and/or information descriptive of an anatomical area shown by the medical images 210.

The exam context information 208 may further comprise interface configuration information, wherein the interface configuration information is descriptive of a geometric layout of the user interface of the medical imaging system 206 and/or information on positions of the exam context information 208 in the screen capture medical image data 202.
The interface configuration information may comprise information identifying the user interface as being associated with a particular vendor of the medical imaging system 206 and/or a vendor of software used to generate the user interface. The control system 200 may thus be enabled to detect a type of user interface based on the interface configuration information. For example, a text information specifying a name and/or version of a software used for generating the user interface may be displayed on the user interface.

Furthermore, the exam context information 208 may comprise exam progress information. In one example, the exam progress information comprises a numerical value indicating a progress of an ongoing medical imaging examination at the medical imaging system 206. The exam progress information may be based on a time since a start of the medical imaging examination, in particular relative to a predetermined duration of the medical imaging examination. In another example, the exam progress information may be based on a number of acquired images during relative to a total number of images to be acquired during the medical imaging examination. The exam context information may be displayed on the user interface.

For example, the medical scanning technique may comprise an MRI examination and the medical imaging system 206 may comprise an MRI imaging system 206, the exam parameters may comprise a magnetic field strength, pulse sequence information about time points and/or durations of magnetic pulses applied by the medical imaging system 206, such as an image sequence, for example a fluid-attenuated inversion recovery (FLAIR), diffusion-weighted imaging (DWI) or gradient echo (GRE) sequence, repetition time between successive pulses, a flip angle at which a magnetization vector is rotated by the pulses, and/or a bandwidth of frequencies collected by a receiver coil of the MRI imaging system 206.

In another example, the medical scanning technique comprises a CT examination and the medical imaging system 206 comprises a CT imaging system 206. In this example, the exam parameters may comprise a tube voltage of an x-ray tube of the CT imaging system 206, a tube current and/or exposure time, a scan mode, for example an axial scan mode or a helical scan mode, a dose modulation parameter of an automatic exposure control (AEC) system, a window level parameter for adapting a brightness of the medical images 210, and/or a window width parameter for adapting a contrast of the medical images 210.

Furthermore, the exam parameters may comprise parameters that are independent of the medical scanning technique, for example a total number of medical images 210 to be acquired, a slice thickness, a slice spacing, a data processing algorithm for reconstructing the medical images 210 from raw data obtained by the medical imaging system 206, a field of view indicated a size of an area to be imaged, a required spatial resolution and/or a temporal resolution of the medical images 210. The exam parameters as well as possible values of the exam parameters may differ depending on the medical scanning technique, and/or hardware capabilities of the medical imaging system 206.

In one example, the extracting of the exam context information 208 comprises extracting text information from the screen capture medical image data 202 using optical character recognition and/or using a neural network, wherein the neural network is configured to detect the text information.

For example, the extracting may comprise extracting the exam progress information using computer vision techniques, for example using optical character recognition to extract the numerical value or using object recognition algorithms to detect an element of the user interface indicating the progress, such as a progress bar.

In one example, the medical images 210 are embedded in the screen capture medical image data 202 as displayed images 210. According to this example, the extracting of the medical images 210 comprises extracting the displayed images 210 as the medical images 210. The extracting further comprises generating the control signal 216. The control signal 216 may comprise a command instructing the user interface of the medical imaging system 206 to switch the user interface into an image view mode, wherein the image view mode is configured to display the medical images 210 as the displayed images 210. The control signal 216 may further comprise instructions for removing elements of the user interface occluding the displayed images 210. For example, a mouse click command may be used to close a popup notification occluding the displayed images 210. Furthermore, the control signal 216 may comprise instructions for iterating through a set of medical images 210, displaying at least a part of at least one medical image 210 of the set of medical images 210 as the displayed images 210 in each iteration. For example, the set of medical images 210 may comprise images of slices of an MRI examination, wherein the control signal 216 comprises interface control commands, wherein the interface control commands comprise mouse movement commands and a series of mouse scrolling commands, resulting in a displaying of the images of slices by executing the series of mouse scrolling commands. Moreover, the control signal 216 may comprise instructions for displaying the displayed images 210 with a higher resolution, a modified contrast and/or a modified brightness, and extracting the images displayed with the higher resolution, the modified contrast and/or the modified brightness as the medical images. In one example, the control signal 216 comprises interface control commands, wherein the interface control commands comprise mouse movement commands and mouse click commands, resulting in mouse clicks being executed on preview images of the medical images 210, thereby displaying the displayed images 210 with a higher resolution.

In one example, the selecting of the image evaluation protocol 212 comprises a selecting of a subset of suitable image evaluation protocols from a set of image evaluation protocols 212, wherein the disclosed method further comprises determining the subset. The determining of the subset may comprise restricting the set of image evaluation protocols 212 to the subset, wherein for the image evaluation protocols of the subset the respective image metric 214 of the image evaluation protocol 212 is applicable to the medical images 210 based on the exam type information.

Said restricting to the subset may further be performed based on the exam context information 208. For example, in case the exam context information 208 comprises text information specifying a protocol name as "MS lesions", the image evaluation protocols 212 may be restricted to image evaluation protocols 212 suitable for processing medical images 210 of the brain. The control system 200 may store information relating a set of image evaluation protocols 212 to medical scanning techniques and/or anatomical areas of the body of the subject to which said image evaluation protocols 212 may be applied. Furthermore, metadata of the medical images 210 extracted along with the medical images 210 themselves may be used to perform the restricting of the set of image evaluation protocols 212.

In one example, the executing of the image evaluation protocol 212 further comprises detecting the image defects by comparing the at least one value of the at least one image metric 214 to at least one threshold value 402 of the image metric 214. The threshold value 402 may be a predefined threshold value, or a dynamically determined threshold value. In particular, the threshold value may be dynamically adapted based on the exam parameters, for example based on the spatial and/or temporal resolution of the medical images 210.

The correction instructions 404 may exemplarily instruct the local operator to repeat the medical imaging examination using identical exam parameters, for example if the detected image defects comprise motion artifacts, or are otherwise unrelated to technical processes executed by the medical imaging system 206 itself. Alternatively, the correction instructions 404 may instruct the local operator to repeat the medical imaging examination using the modified values of the exam parameters or to perform a different type of medical imaging examination. For example, if the medical imaging examination results in medical images 210 of the brain of the patient and the exam context information 208 includes the protocol name "MS lesions", the image evaluation protocol 212 may be chosen to evaluate a sufficient coverage of the brain among the at least one image metric 214. The threshold value 402 for the coverage may exemplarily be set to 98%. In this example, the image evaluation protocol 212 may provide a value for the coverage indicating that 90% of the brain is covered by the medical images 210. As this coverage is below the threshold value 402 for sufficient coverage, the control signal 216 will notify the local operator of the image defect of insufficient coverage. The control signal 216 may further comprise instructions for moving the subject relative to the medical imaging system 206, such that a repeated medical imaging examination after said moving of the subject may result in an improved value for the coverage.

The control signal 216 may further result in a visual signal such as a flashing light and/or an audio signal such as synthetic speech or an alarm sound being provided at the site of the medical imaging system 206 and/or the site of the (remote) operator in response to the detecting of the image defects. Furthermore, the control signal 216 may provide a pop-up notification on the display unit 613" at the site of the operator and/or a pop-up notification on a mobile device of the computer system 602 of the medical imaging system 206, wherein the mobile device preferably comprises a tablet. In some examples, haptic, audio or any combination of such are provided to the operator of the control system 200.

In one example, the screen capture medical image data 202 is received repetitively over time. According to this example, the executing of the image evaluation protocol 212 is performed based on anyone of the following: the exam progress information, a time period since a previous executing of the image evaluation protocol 212, at least one value of the at least one image metric 214 obtained in the previous executing 108 of the image evaluation protocol 212, a request by an operator of the control system 200.

According to this example, the executing of the image evaluation protocol may be automatically started when the extracted exam progress information reaches predetermined numerical values, for example, when 20%, 40%, 60%, and/or 80% of the medical imaging examination has been completed. Alternatively, other predetermined numerical values may be chosen, in particular depending on a total duration of the medical imaging examination, a size and/or number of the medical images 210 and/or a computational complexity of the image evaluation protocol 212. In a further alternative example, the image evaluation protocol 212 may be executed in fixed time intervals, for example every minute after a previous executing 108 of the image evaluation protocol 212. In yet another alternative example, the operator may send the request to the control system 200, enabling a manual execution of the image evaluation protocol 212 at a time point chosen by the operator. Said manual execution may be performed while the medical imaging examination is ongoing or after the medical imaging examination has been completed.

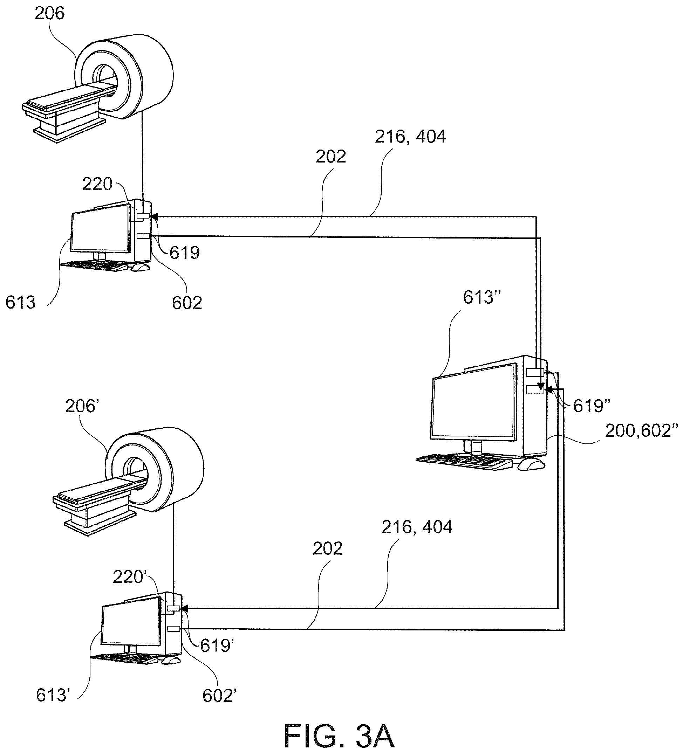

In one example, the extracted medical images 210 are stored in the image database 220 of the control system during the medical imaging examination and loaded from the image database 220 after the medical imaging examination has been concluded to select and execute the image evaluation protocol on the medical images 210. According to this example, the local operator and/or other experts possessing relevant domain knowledge may be enabled to adapt the medical imaging examinations and/or exam parameters, in particular default values of the exam parameters, based on the values of the image metrics 214 obtained by the executing 108 of the image evaluation protocol 212. Thereby, a quality of medical images 210 of future subjects to be imaged using the medical imaging system 206 may be improved independent of a presence of a subject in the medical imaging system 206 at a time at which the image evaluation protocol 212 is executed. Fig. 3A is a schematic of the control system 200 interacting with multiple computer systems 602, 602' of multiple medical imaging systems 206, 206', wherein a remote computer system 602" comprises the control system 200. Any examples described with reference to Fig. 1 and Fig. 2 equally apply to the control system 200 and the multiple medical imaging systems 206, 206' shown in Fig. 3A.

According to the example shown in Fig. 3A, the computer system 602" may comprise a computer system of the (remote) operator. According to this example, the capture modules 218, 218' of the medical imaging systems 206, 206' may capture the screen capture medical image data 202 from the display units 613', 613' of the computer systems 602, 602' of the medical imaging systems 206, 206' as shown in Fig. 2. Said screen capture medical image data 202 may be provided to the control system 200, 602" via the I/O-interfaces 619, 619', 619" to be displayed on the display unit 613" of the computer system 602".

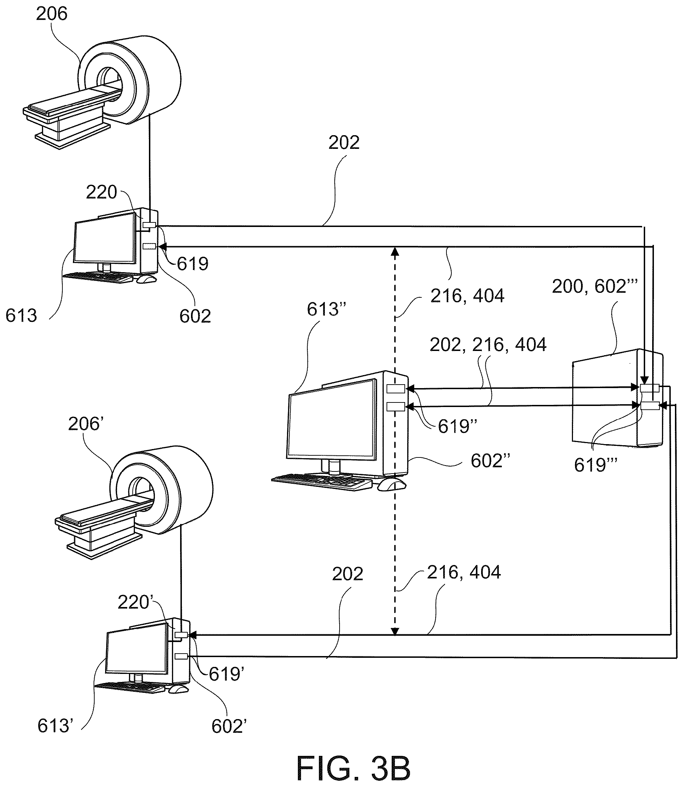

Fig. 3B is a schematic of the control system 200 interacting with multiple computer systems 602, 602' of multiple medical imaging systems 206, 206' and the computer system 602". According to Fig. 3B, a cloud-based computer system 602‴ comprises the control system 200. Any examples described with reference to Fig. 1 and Fig. 2 equally apply to the control system 200 and the multiple medical imaging systems 206, 206' shown in Fig. 3B.

According to the example shown in Fig. 3B, the computer system 602" may comprise a computer system of the (remote) operator. According to this example, the capture modules 218, 218' of the medical imaging systems 206, 206' may capture the screen capture medical image data 202 from the display units 613', 613' of the computer systems 602, 602' as shown in Fig. 2. Said screen capture medical image data 202 may be provided to the control system 200, 602‴ via the I/O-interfaces 619, 619', 619‴ and provided by the control system 200 to the computer system 602" of the remote operator via the I/O interfaces 619", 619‴ to be displayed on the display unit 613" of the computer system 602". According to this example, the disclosed method may be performed by the cloud-based computer system 602‴, for example due to increased computational capacities of the cloud-based computer system 602‴ compared to the computer systems 602, 602', 602", resulting in a faster executing of the image evaluation protocol. The control system 200, 602‴ may be configured to display the user interfaces of the medical imaging systems 206, 206' on the display 613" of the computer system 602", enabling the operator of the computer system 602" to identify image defects. The control system 200, 602" may further provide the control signal 216 comprising the correction instructions 404 to the computer system 602", enabling the operator of the computer system 602" to approve or modify the corrections instructions 404, wherein the computer system 602" provides the control signal 216 comprising the approved or modified corrections instructions 404 to the cloud-based computer system 602‴, wherein the cloud-based computer system 602‴ further provides the control signal 216 comprising the approved or modified correction instructions 404 to the computer systems 602, 602'. Alternatively, the control signal 216 comprising the approved or modified correction instructions 404 may be provided directly from the computer system 602" to the computer systems 602, 602', as indicated by the dashed lines in Fig. 3B.

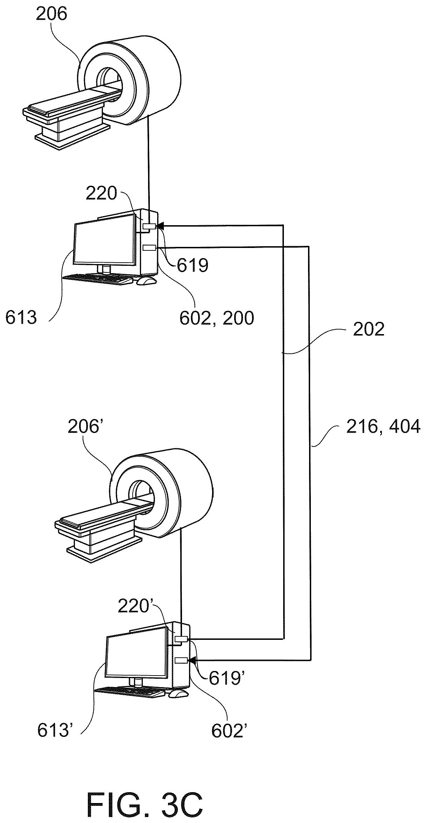

Fig. 3C is a schematic of the control system 200 interacting with multiple computer systems 602, 602' of multiple medical imaging systems 206, 206', wherein the computer system 602 of the medical imaging system 206 comprises the control system 200. Any examples described with reference to Fig. 1 and Fig. 2 equally apply to the control system 200 and the multiple medical imaging systems 206, 206' shown in Fig. 3C. According to the example shown in Fig. 3C, the computer system 602 of the medical imaging system 206 corresponds to the computer system 602" of the operator.

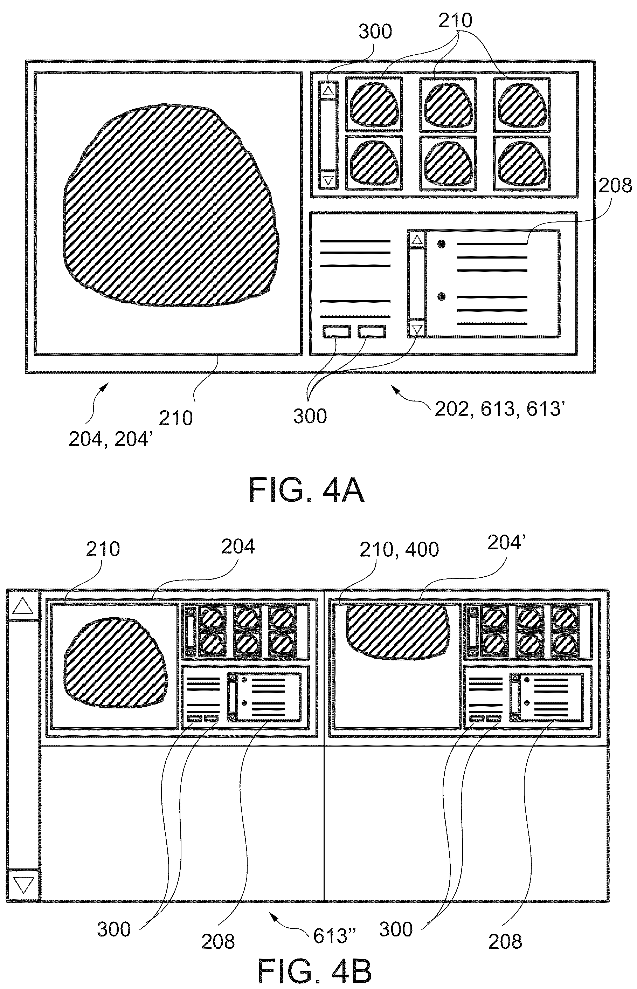

Fig. 4A is a schematic of the user interface 204, as shown on the display unit 613 of the computer system 602 of the medical imaging system 206, as shown in Fig. 3A, Fig. 3B and Fig. 3C. The schematic shown in Figure 4A may equally apply to the user interface 204' of the medical imaging system 206' as shown on the display unit 613' of the medical imaging system 206' as shown in Fig. 3A, Fig. 3B and Fig. 3C.

The control functionalities may comprise interface control commands, wherein the interface control commands are configured to interact with controllable elements 300 of the user interface 204. The controllable elements 300 exemplarily comprise buttons, toggles, drop-down menus, text fields for receiving text input, and/or scroll bars. According to Fig. 4A, the user interface 204 is shown to display a set of medical images 210 as preview images in an upper right side of the user interface 204, controllable elements 300 in the upper right side and a lower right side of the user interface 204, and a particular medical image 210 displayed as a high resolution medical image 210 on a left side of the user interface 204. The medical images 210 and/or controllable elements 300 may alternatively be arranged at different locations of the user interface 204.

Fig. 4B is a schematic of screen replications of multiple user interfaces 204, 204' of multiple medical imaging systems 206, 206' displaying medical images 210 as described in Fig. 4A. Said screen replications may be shown on the display unit 613" of the (remote) operator in accordance with the examples of Fig. 3A or Fig. 3B. Alternatively, in accordance with the examples of Fig. 3C, the screen replications shown in Fig. 4B may equally be shown on the display unit 613 of the computer system 602.

The capture module 218 as shown in Fig. 2 may capture the user interfaces 204, 204' as displayed on the display units 613, 613' of the multiple medical imaging systems 206, 206'. The control system 200 may further display the multiple user interfaces 204, 204' on the display unit 613" of the computer system 602". The multiple user interfaces 204, 204' may be displayed in various layouts, for example in a grid, wherein the grid may exemplarily comprise a 2x1, a 3x1, or a 2x2 layout as shown in Fig. 4B. Entries of the command database as shown in Fig. 2 may further comprise information on positions of controllable elements 300, the exam context information 208 and/or displayed images 210 for different positions of a particular user interface 204, 204' in the grid. Alternatively, the positions of the controllable elements 300, the displayed images 210 and/or the exam context information 208 in the grid may be determined using a trained object detection model. Said object detection model may for example comprise a neural network trained on labeled images of the grid, wherein the labels comprise information specifying the positions of the controllable elements 300, the exam context information 208 and/or the displayed images 210 in a specific grid, for example a 2x2 grid.

Fig. 4B further shows an exemplary image defect 400 in the screen replication of the user interface 204', wherein an anatomical structure imaged by the medical imaging system 206' shows insufficient coverage in a medical image 210 acquired by the medical imaging system 206'. In response to detecting said image defect 400, the control system 200 may send the control signal 216 comprising the correction instructions 404 to the computer system 602' of the medical imaging system 206'. According to this example, the correction instructions 404 may comprise instructions for adapting the field of view of the medical imaging system 206' or moving the subject imaged by the medical imaging system 206'.The control system 200 may exemplarily enable a concurrent execution of the control functionalities using the control signal 216 on any one of the user interfaces 204, 204'.

The examples described with regards to Fig. 4A and Fig. 4B may equally apply to implementations of the control system 200 at the site of the (remote) operator as described in Fig. 3A, implementations of the control system 200 in a cloud-based computing system 602‴ as described in Fig. 3B and implementations of the control system 200 at the local site of the medical imaging system 206 as described in Fig. 3C.

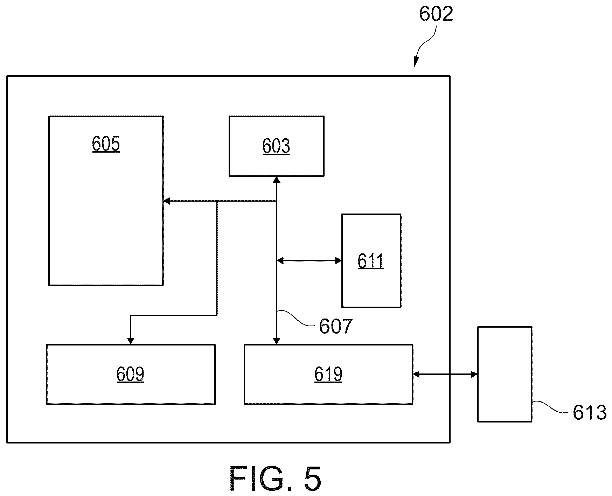

Fig. 5 is a block diagram of an exemplary computer system 602 for implementing the present method in accordance with an example of the present subject matter. The components of the computer system 602 as described in relation to Fig. 5 may equally apply to components of further computer systems 602', 602" and 602‴.

The components of the computer system 602 may include, but are not limited to, one or more processors or processing units 603, a storage system 611, a memory unit 605, and a bus 607 that couples various system components including memory unit 605 to processor 603. The storage system 611 may include for example a hard disk drive (HDD). The memory unit 605 may include computer system readable media in the form of volatile memory, such as random access memory (RAM) and/or cache memory.

The computer system 602 may also communicate with one or more external devices such as a keyboard, a pointing device, a display unit 613, etc.; one or more devices that enable a user to interact with computer system 602; and/or any devices (e.g., network card, modem, etc.) that enable the computer system 602 to communicate with one or more other computing devices. Such communication can occur via I/O interface(s) 619. Still yet, the computer system 602 can communicate with one or more networks such as a local area network (LAN), a general wide area network (WAN), and/or a public network (e.g., the Internet) via a network adapter 609. As depicted, the network adapter 609 communicates with the other components of the client system 602 via bus 607.

The memory unit 605 is configured to store applications that are executable on the processor 603. For example, the memory unit 605 may comprise an operating system as well as one or more application programs. The application programs comprise instructions that when executed enable to perform the method described with reference to Fig. 1.

As will be appreciated by one skilled in the art, aspects of the present invention may be embodied as an apparatus, method, computer program or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon. A computer program comprises the computer executable code or "program instructions".

The term "computer system" refers to data processing hardware and encompasses all kinds of apparatus, devices, and machines for processing data, including by way of example, a programmable processor, a computer, or multiple processors or computers. The apparatus can also be or further include special purpose logic circuitry, e.g., a central processing unit (CPU), a FPGA (field programmable gate array), or an ASIC (application specific integrated circuit). In some implementations, the data processing apparatus and/or special purpose logic circuitry may be hardware-based and/or software-based. The apparatus can optionally include code that creates an execution environment for computer programs, e.g., code that constitutes processor firmware, a protocol stack, a database management system, an operating system, or a combination of one or more of them. The present disclosure contemplates the use of data processing apparatuses with or without conventional operating systems, for example LINUX, UNIX, WINDOWS, MAC OS, ANDROID, IOS or any other suitable conventional operating system.

Any combination of one or more computer readable medium(s) may be utilized. The computer readable medium may be a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device.

'Computer memory' or 'memory' is an example of a computer-readable storage medium. Computer memory is any memory which is directly accessible to a processor. 'Computer storage' or 'storage' is a further example of a computer-readable storage medium. Computer storage is any nonvolatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly.

Generally, the program instructions can be executed on one processor or on several processors. In the case of multiple processors, they can be distributed over several different entities. Each processor could execute a portion of the instructions intended for that entity. Thus, when referring to a system or process involving multiple entities, the computer program or program instructions are understood to be adapted to be executed by a processor associated or related to the respective entity.

It is understood that one or more of the aforementioned examples may be combined as long as the combined examples are not mutually exclusive.

While the invention has been illustrated and described in detail in the drawings and foregoing description, such illustration and description are to be considered illustrative or exemplary and not restrictive; the invention is not limited to the disclosed examples.

### REFERENCE SIGNS LIST

- 100: method for performing image quality checks
- 102, 104, 106, 108, 110: method steps
- 104: extraction module
- 200: control system
- 202: screen capture medical image data
- 204 , 204': user interface
- 206, 206': medical imaging system
- 208: exam context information
- 210: medical images, displayed images
- 212: image evaluation protocol, image evaluation module
- 214: (image) metric
- 216: control signal
- 218, 218': capture module
- 220: image database
- 300: controllable elements
- 302: interface control command generator
- 304: command database
- 400: image defects
- 402: threshold value
- 404: correction instructions
- 602, 602', 602", 602‴: computer system
- 603: processors
- 605: memory unit
- 607: bus
- 609: network adapter
- 611: storage system
- 613, 613', 613": external devices, display unit
- 619, 619', 619", 619‴: I/O-Interface

## Claims

1. A computer-implemented method (100) for performing image quality checks using a control system (200), the method comprising:
- receiving (102) by the control system (200) screen capture medical image data (202) of a user interface (204) of a medical imaging system (206);
- extracting (104) exam context information (208) and medical images (210) from the screen capture medical image data (202);
- based on the extracted exam context information (208), selecting (106) an image evaluation protocol (212), wherein the image evaluation protocol (212) comprises at least one image metric (214);
- executing (108) the image evaluation protocol (212) on the extracted medical images (210), resulting in at least one value of the at least one image metric (214); and
- based on the at least one value of the at least one image metric (214), providing (110) a control signal (216) to the medical imaging system (206).

2. The method (100) according to claim 1, wherein a capture module (218) is configured for receiving (102) of screen captured medical image data (202), the capture module (218) providing a screen replication of the user interface (204) of the medical imaging system (206).

3. The method (100) according to claim 1 or 2, wherein the control system (200) is further configured for providing control functionalities to the user interface (204) of the medical imaging system (206), wherein the extracting (104) of the exam context information (208) and/or the medical images (210) from the screen capture medical image data (202) is repeatedly performed using the control signal (216).

4. The method (100) according to any one of the preceding claims, wherein the exam context information (208) comprises any one of the following:
- exam type information, wherein the exam type information is descriptive of a medical scanning technique that was used for generating the medical images (210), and/or information descriptive of an anatomical area shown by the medical images (210);
- interface configuration information, wherein the interface configuration information is descriptive of a geometric layout of the user interface (204) of the medical imaging system (206) and/or information on positions of the exam context information (208) in the screen capture medical image data (202);
- exam progress information;
- exam parameters, wherein the exam parameters comprise medical imaging system parameters of the medical scanning technique used for generating the medical images (210).

5. The method (100) according to any one of the preceding claims, wherein the control system (200) further comprises an interface control command generator (302) configured to generate the control signal (216), wherein the control signal (216) comprises any one of the following: a mouse movement command, mouse click command, mouse wheel scrolling command, a keystroke on a keyboard, a text copy command, a text insertion command and/or a screenshot command.

6. The method (100) according to any of the preceding claims, wherein the extracting (104) of the exam context information (208) comprises extracting text information from the screen capture medical image data (202) using optical character recognition and/or using a neural network, wherein the neural network is configured to detect the text information.

7. The method (100) according to any one of the preceding claims, wherein the medical images (210) are embedded in the screen capture medical image data (202) as displayed images, and wherein the extracting (104) of the medical images (210) comprises generating the control signal (216) comprising a command instructing the user interface (204) of the medical imaging system (206) to perform any one of the following:
- switch the user interface (204) into an image view mode, wherein the image view mode is configured to display the medical images (210) as the displayed images,
- remove elements of the user interface (204) occluding the displayed images,
- iterate through a set of medical images, displaying at least a part of the medical images (210) of the set of medical images as the displayed images in each iteration,
- display the displayed images with a modified resolution, a modified contrast and/or a modified brightness, and extracting the images displayed with the modified resolution, the modified contrast and/or the modified brightness as the medical images.

8. The method (100) according to any one of claims 4 to 7, wherein the selecting (106) of the image evaluation protocol (212) comprises a selecting of a subset of suitable image evaluation protocols from a set of image evaluation protocols (212), the method further comprising determining such subset, the determining comprising at least anyone of the following:
- restricting the set of image evaluation protocols (212) to the subset, wherein for the image evaluation protocols of the subset the respective metric (214) of the image evaluation protocol (212) is applicable to the medical images (210) based on the exam type information,
- restricting the set of image evaluation protocols (212) to the subset, wherein for the image evaluation protocols of the subset the respective metric (214) of the image evaluation protocol (212) specifies required exam parameters, wherein the exam parameters of the exam context information (208) satisfy the required exam parameters,
- restricting the set of image evaluation protocols (212) to the subset based on an average accuracy, an average runtime and/or a computational complexity of the image evaluation protocol.

9. The method (100) according to any one of claims 3 to 8, wherein the executing (108) of the image evaluation protocol (212) further comprises:
- detecting image defects (400) by comparing the at least one value of the at least one image metric (214) to at least one threshold value (402) of the image metric (214) and
- in response to the detecting of the image defects (400), determining correction instructions (404), wherein the correction instructions (404) are configured for minimizing the occurrence of image defects (400), and preferably wherein the correction instructions (404) comprise positioning instructions for positioning a subject imaged by the medical imaging system (206) and/or modified values of the exam parameters.

10. The method (100) according to claim 9, wherein the control signal (216) is configured for instructing the user interface (204) of the medical imaging system (206) for executing the correction instructions (404), wherein preferably the correction instructions (404) are provided automatically after the executing (108) of the image evaluation protocol (212).

11. The method (100) according to any one of claims 4 to 10, wherein the screen capture medical image data (202) is received (102) repetitively over time, wherein the executing (108) of the image evaluation protocol (212) is performed based on anyone of the following: the exam progress information, a time period since a previous executing (108) of the image evaluation protocol (212), at least one value of the at least one image metric (214) obtained in the previous executing (108) of the image evaluation protocol (212), a request by an operator of the control system (200).

12. The method (100) of any one of the preceding claims, wherein the medical imaging system (206) is any one of the following: a magnetic resonance imaging system, a computed tomography system, a positron emission tomography system, an angiography system, a single photon emission tomography system, a digital fluoroscopy system, a diagnostic ultrasound system.

13. A control system (200),
- the control system (200) configured to carry out any one of the method claims 1-12.

14. The control system according to claim 13, wherein the control system is configured to perform image quality checks.

15. A computer program comprising machine executable instructions configured for causing a control system (200) to perform the method (100) of any one of claims 1 to 12.
